## Europäisches Patentamt
## European Patent Office
## Office européen des brevets

(19)

(11) Veröffentlichungsnummer: **0 065 482**
**B2**

(12) ## NEUE EUROPÄISCHE PATENTSCHRIFT

(45) Veröffentlichungstag der neuen Patentschrift:
**24.10.90**

(51) Int. Cl.⁵: **A 61 F 2/34**

(21) Anmeldenummer: **82730068.2**

(22) Anmeldetag: **18.05.82**

(54) Künstliche Hüftpfanne.

(30) Priorität: **18.05.81 DE 3120148**
**27.11.81 DE 3147707**
**15.03.82 EP 82730035**

(43) Veröffentlichungstag der Anmeldung:
**24.11.82 Patentblatt 82/47**

(45) Bekanntmachung des Hinweises auf die
Patenterteilung:
**05.02.86 Patenblatt 86/06**

(45) Bekanntmachung des Hinweises auf die
Entscheidung u̇ber den Einspruch:
**24.10.90 Patentblatt 90/43**

(84) Benannte Vertragsstaaten:
**AT BE CH DE FR GB IT LI NL SE**

(56) Entgegenhaltungen:
DE-A-2 323 456        DE-A-2 839 661
DE-A-2 411 617        FR-A-2 099 259
DE-A-2 645 101        FR-A-2 295 729

Firmenprospekt "La prothèse universelle de
Lord à appui trochantéro - diaphysaire
Madréporique" der Firma s.a. benoist girard &
Cie.

(73) Patentinhaber: **MECRON MEDIZINISCHE
PRODUKTE GMBH**
**Nunsdorfer Ring 23-27**
**D-1000 Berlin 48 (DE)**

(72) Erfinder: **Goymann, Volkmar, Prof. Dr. med.**
**Forstmannstrasse 56**
**D-4300 Essen 16 (DE)**

(74) Vertreter: **Christiansen, Henning, Dipl.-Ing.**
**Patentanwalt CHRISTIANSEN Pacelliallee 43/45**
**D-1000 Berlin 33 (DE)**

(56) References cited:
**Firmenprospekt IP Produkt Mitteilung
"Zementlose totale Hüftgelenkprothese -
Schraubsystem - nach Reimer" der Firma IP
interplanta GmbH.**

**Firmenprospekt "Lord Madreporische Prothese
ohne Zement" der Firma Howmedica GmbH.**

**Sonderdruck aus Biomedizinische Technik 32
(1987) 12, S.299-305 "Untersuchung der primär
postoperativen Spannungsverteilung im
Acetabulum nach Implantation von
zementfreien Schraubpfannen" Kranz/Dietrich.
Orthop. Praxis 6/86, S. 473-480 "Experimentelle
Untersuchungen des Implantationsverhaltens
und der primären Festigkeit der Mecring-
Hüftendoprothesenpfanne bei technisch
verschiedenen Implantationsweisen" Löer et al.**

## Beschreibung

Die Erfindung betrifft ein in den Knochen einschraubbares Trägerelement einer künstlichen Hüftpfanne mit Einsatz wie im Oberbegriff des Anspruchs 1 angegeben. In dem Firmenprospekt "LA PROTHESE UNIVERSELLE DE LORD A APPUI TROCHANTERO-DIAPHYSAIRE MADREPORIQUE" der Firma S. A. Benois Girard & Cie, aus 1979 ist eine derartige Hüftpfanne mit konvex geformter, ein Gewinde aufweisender Außenoberfläche beschrieben. Diese Hüftpfanne ist nach dem Vorschneiden des Gegengewindes im Acetabulum nur in einer vorgegebenen Richtung bis in eine Position einschraubbar, wie sie vom Gewindeschneider bereits einmal erreicht worden ist.

Des weiteren ist aus der DE—A—26 45 101 eine Hüftpfanne mit Kugelkalottenförmiger Gestalt bekannt, die im unteren Bereich ihres Außenmantels mit einem selbstschneidenden Gewinde versehen ist. Da bei dieser Pfanne das Gewinde in die kalottenförmige Grundform eingepreßt oder sonstwie eingearbeitet ist, ist die volle Einschraubbarkeit in das Acetabulum nur dann möglich, wenn dieses entsprechend vorbereitet wurde. Eine Kontrolle des vollständigen Einschraubens der Pfanne ist nicht möglich. Das ist insbesondere deshalb nachteilig, weil beim selbstschneidenden Eindrehen nicht ein plötzlicher Anstieg des Einschraubmoments beim Erreichen der Endposition, in der das Pfannendach am Acetabulum anliegt, festzustellen ist.

Ein weiteres Trägerelement ist aus der DE—A—24 11 617 bekannt, mit dem eine Gelenkfläche einschnappbar verbunden ist, wobei hier die Tragrippen an einer Außenfläche zwar gewindeförmig angeordnet sind und die Außenfläche der Mantelfläche eines Kegelstumpfs entspricht, das Trägerelement ist aber lediglich in einer einzigen Richtung einschraubber, die durch die Form der zuvor in den Knochen angebrachten Ausfräsung bestimmt ist.

Weiterhin ist ein Trägerelement aus der FR—A—20 99 259 bekannt. Bei der dort insbesondere in Figur 12 dargestellten Ausführung ist nachteilig, daß durch die Anbringung des Gewindes an einem auf die höchste Erhebung aufgesetzten Zentrierstiftes relativ viel Knochensubstanz beschädigt wird und weiterhin die Anbringung nur in einer einzigen vorgegebenen position möglich ist, welche bereits beim Herstellen der Ausparung für das Trägerelement im Hüftknochen festgelegt sein muß.

Vielfach ist es aber erwünscht bzw. sogar notwendig, noch nach dem Erzeugen der die Hüftpfanne oder deren Trägerelement aufnehmenden Ausfräsung eine Korrektur hinsichtlich der Position, d.h. Ausrichtung der den Schaftprothesenteil aufnehmenden Gelenkpfanne vorzunehmen.

Der in Anspruch 1 angegebenen Erdindung liegt die Aufgabe zugrunde, ein Trägerelement zu schaffen, welches nach dem Herstellen der fertigen Ausfräsung derart unterschiedlich eingebracht werden kann, daß dem Chirurgen zum einen die Auswahl von besonders tragfähigen Knochenbereichen und zum anderen eine gewisse Ausrichtung der Bezugsrichtung der künftigen Hüftgelenkprothese durch die beim Einschrauben des Gewindes gewählte Richtung möglich ist.

Die Erfindung beruht auf der Erkenntnis, daß die Krafteinleitung in den Beckenknochen möglichst in Richtung der bei Belastung der Prothese auftretenden Kräfte erfolgen sollte, was einerseits durch die oberen Bereiche des verrundeten Trägerelements und bei ringförmiger Ausbildung desselben über das durch den Ring hindurchragenden Pfannenteil erfolgen kann. Die Fixierung der Pfanne durch Gewindesegmente des Trägerelements konzentriert sich auf den Kortikalisbereich.

Das Einsetzen des Trägerelementes ist dadurch vereinfacht daß das Gewindeprofil das notwendige Gegengewinde beim Einschrauben selbst erzeugt, so daß ein vorheriges Gewindeschneiden überflüssig ist.

Dadurch, daß in dem zunächst separat in den Beckenknochen einzuschraubenden Trägerelement eine runde Aussparung vorgesehen ist, deren Durchmesser bevorzugt größer aus gebildet ist als die Höhe des verbleibenden Rings, ist der Durchblick auf den Boden der gefrästen Aussparung freigegeben, so daß beim Einschrauben ohne zusätzliche Hilfsmittel leicht beurteilt werden kann, wann das Trägerelement seine Endposition erreicht hat.

Die ringförmige Gestaltung erfolgt günstig derart, daß die sich ergebende Elastizität in bezug auf Verformungen des Rings derjenigen des diesen umgebenden Knochenbereichs entspricht, so daß eine weitgehende Homogenität des eingesetzten Ringes und des Beckenbereichs besteht und damit die Einleitung von Belastungskräften stets großflächig erfolgt. Damit sind auch die Auswirkungen des Kerbverhaltens des Gewindes und die Gefahr von Knochenresorption verrin gert. Der Pfanneneinsatz weist dazu in Bezug auf das Trägerelement hinsichtlich seines Durchmessers ein gewis ses Spiel auf, so daß die auftretenden Belastungskräfte weitgehend ohne Verformung des Einsatzes aufgenommen werden können.

Im Sinne einer optimalen Krafteinleitung in das Becken ist die Anlagefläche Einsatz/Trägerelement senkrecht zur bevorzugt auftretenden Kraft, d.h. beispielsweise um ca. 45° zur Einschraubachse und zwar konzentrisch um diese herum, angeordnet. Die konische Form bringt den Vorteil mit sich, daß der Einsatz verdrehungssicher in dem Ring stekt und daß die Kräfte, insbesondere die axial wirkenden Kräfte, nicht nur von einem Kragen des Einsatzes son dern auch von der konischen Wand des Einsatzes aufgenommen werden.

Übliche selbstschneidende Gewindeprofile, wie sie für normale Knochenschauben verwendet werden, sind für die Anbringung auf einem balligen Körper ungeignet, da nicht gewährleistet ist, daß wie bei zylindrischen oder konischen Gewindeschneidern das einer Schneidnut benachbarte beim Einschrauben voran geführte Kantenende eines Gewindesegments auch zuerst mit den noch nicht mit einem Gewinde versehenen Bereichen der Knochenoberfläche in Berührung kommt.

Das hat seine Ursache darin, daß in der Ebene des Einstechens der Gewindesgmente in die Knochenoberfläche der Durchmesser der gedachten, die Gewindesegmente tanglerenden Halbkugel größer ist als der entsprechende Durchmesser der im Knochen halbkugelförming ausgefrästen Bereiche. Beim Ineinanderdringen der beiden Halbkugeloberflächen nimmt der Abstand der beiden Kugeloberflächen von deren Schnittpunkt in Einschraub richtung stark zu, so daß die erstmalige Berührung zwischen Knochenoberfläche und den sich einschneidenden Gewindesegementen nicht notwendigerweise am vorderen Kantenende bei der Schneidnut, sondern eher auf dem "Rucken" des Gewindesegments stattfindet. Beim Eisnchneiden mit den "Rücken" der Gewindeflanken sind die dabei aufzubringenden Kräfte aber dermaßen groß, daß ein "Selbstschneiden" des Gewindes durch das Trägerelement praktisch ausgeschlossen ist. Ein Einbringen ohne Vorschneiden des Gewindes ist damit in der Praxis nur möglich, wenn die Gewindeflanken die in Anspruch 4 angegebene Form haben, so daß sie beim erstmaligen Berühren der Knochenwand eine möglichst punktförmige Berührung erzeugen und in die Knockenoberfläche einstechen. Die Rückflanke kann dann den bei Schniedwerkzeugen notwendigen "Freiwinkel" aufweisen. Diese Form der Gewinde segmente wird bevorzugt dadurch erreicht, daß die in Drehrichtung beim Eins Schrauben zurückliegenden Bereiche der höchsten Erhebungen der Gewindesegmente zunehmend verringert sind, was durch ein entsprechendes Abtragen der Gewindesegmente durch Verrunden oder durch Fräsern erfolgen kann.

Mit dieser von der normalen Form eines Knochengewindes abweichenden Gestaltung der Gewindesegmente ist weiterhin der Vorteil verbunden, daß bei bevorzugt abgestumpfter Gestaltung der in Drehrichtung beim Einschrauben zurücklie genden Bereiche die Stabilität der Position des Tragelementes im implantierten Zustand heraufgesetzt ist, da die Kerbwirkung herabgesetzt ist und damit durch die bei Verformung des Knochens auftretenden Kräfte nicht zu einem tieferen Eindringen und damit einer Auslockerung des Trägerelementes führen können.

Die ringförmige Gestaltung hat weiterhin den Vorteil, daß die Elastizität des Trägerelementes durch Werkstoffwahl und Bemessung derjenigen der umgebenen Knochenbereiche weitgehend angepaßt sein kann, so daß das Element bezüglich der zu übertragenden Kräfte keinen Fremdkörper darstellt und die Krafteinleitung über möglichst große Flächenbereiche verteilt erfolgt. Insoweit ist es weiterhin günstig, wenn die in das Tragelement einzusetzende Pfanne, die vorzugsweise aus Kunststoff gefertigt ist, mit in verformbaren Bereichen in das Trägerelements in Kontakt kommenden Teilen ein gewisses Spiel aufweist, wobei die Krafteinleitung von der Prothese her über entsprechende Anschlagflächen zum Trägerelement bzw, in den Knochen er folgt.

Durch die ringförmige Ausbildung des Trägerlementes werden einerseits diejenigen Knochenbereiche, welche eine maximale Kraftaufnahme gestatten, bevorzugt zur Krafteinleitung in bezug auf Querkräfte herangezogen, so daß eine optimale Haltewirkung vorhanden ist, während die von der Prothese übertragenen Druckkräfte durch den Ring hin durch auf den Pfannenboden und den Pfannenboden direkt in den Knochen eingeleitet werden, worzu es einer Haltewirkung durch ein Gewinde oder dergleichen nicht bedarf.

Bei einem an das erfindungsgemäße Trägerelement angepaßten System von in dieses einzusetzenden Pfannenteilen kann der Chirurg nach dem Einschrauben des Elementes durch Auswahl eines spezifisch geeigneten Einsatzes noch Korrekturen bezüglich der späteren Lage des künftigen Drehpunkts der Prothese bewirken. Dazu wird beispielsweise mittels einer geeigneten sterilen Meßvorrichtung die gewünschte Lage des Drehpunkts und die axiale Ausrichtung der Ausnehmung zur Aufnahme des Prothesenkopfes ermittelt und ein entsprechend ausgesuchtes Pfannenteil eingesetzt, welches die gewünschten Maße aufweist. Ein System von an das erfindungsgemäße Trägerelement angepaßten Pfannene insätzen weist demnach unterschiedliche Höhen oder radial versetzte Dreh punkte für den Femurprothesenteil auf, wobei Kombinationen der vorgenannten zu verändernden Größen in einem einzigen Einsatzteil möglich sind. Eine Ausrichtung der Neigung der Bezugsachse erfolgt durch ein entsprechend geneigtes Einschrauben des Trägerelements selbst, so daß insofern kein nachträglicher Ausgleich durch das einzusetzende Pfannenteil notwendig ist.

Das ringförmige Trägerelement besteht bevorzugt aus Titan, während die Pfanneneinsätze aus Kunststoff hergestellt sind. Damit ergibt sich der Vorteil, daß die kostengünstig herstellbaren Einsätze ohne weiteres in verschiede nen Varianten vorrätig gehalten werden können, während das Trägerelement jeweils pro Außendurchmesser nur einmal am Lager gehalten werden muß, um allen auftretenden Fällen gerecht zu werden. Pfanneneinsatz und Trägerelement bilden insgesamt einen halbkugelförmigen Einsatz.

Andere bevorzugte Weiterbildungen sínd in den abhängigen Ansprüchen angegeben. Ein vorteilhafts Ausführungsbeispiel ist in den Figuren dargestellt und wird nachfolgend näher beschrieben. Es zeigen:

Figur 1 ein Ausführungsbeispiel des erfindungsgemäßen Trägerelementes mit eingesetztem Pfannenteil in Seitenansicht,

Figur 2 das in Figure 1 dargestellte Trägerelement in perspektivischer Darstellung schräg von unten gesehen,

Figur 2a eine Darstellung eines Details gemäß Figur 2,

Figur 3 ein Pfanneneinsatz für das in Figur 2 dargestellte Trägerelement, ebenfalls in perspektivischer Ansicht,

Figur 4 eine Schnittdarstellung eines in Trägerelement einsetzbaren Einsatzes,

Figur 5 eine Schnittdarstellungen eines in eines Knochenexkavitation eingesetzten Trägerelements,

Figur 6 eine Schnittdarstellung des Trägerelementes gemäß Figur 5 während des Einschraubvorgangs sowie

Figur 7 eine Dartstellung der geometrischen Verhältnisse bei Figur 6 in vergrößerter Wiedergabe.

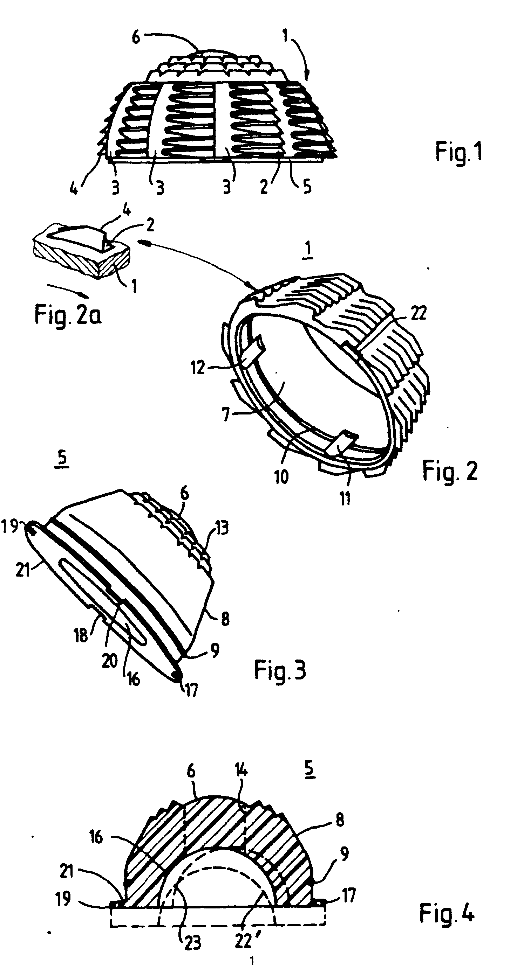

Bei dem in Figur 1 dargestellten Trägerelement 1, welches ringförmig ausgebildet ist und dessen Außenfläche der Oberfläche einer Kugelzone entspricht, sind eine Anzahl von geneigt angeordneten Gewindesegmenten 2 vorgesehen, welche durch Schneidnuten 3 voneinander getrennt sind. Die Gewindesegmente sind an ihren beim Einschrauben vorangeführten Teilen scharfkantig ausgeführt und stehen hier am weitesten von der Kugeloberfläche ab. Sie bilden im Bereich der Schneidnuten scharfkantige Spitzen, welche beispielhaft mit 4 bezeichnet sind. In bezogen auf die beim Einschrauben aufzuwendende Drehrichtung weiter zurückliegenden Bereichen sind die Gewindesegmente zu ihren Außenkanten abgestumpft bis abgeflacht ausgebildet, was durch eine parallele Bearbeitung der übereinanderliegenden Gewindesegmente mittels Fräsen oder dgl. erfolgen kann. Das ringförmige Trägerelement besteht bevorzugt aus Titan, dessen Elastizitätseigenschaften bei geeigneter Querschnittsbemessung in etwa denen des umgebenden Knochenmaterials entspricht.

Ein Pfanneneinsatz 5 ist an die inneren Querschnittsabmessungen des Trägerelementes 1 angepaßt und läßt sich in das bereits in den Knochen eingeschraubte Trägerelement von unten her einsetzen, wobei der obere kuppelförmige Bereich 6 des Einsatzes das Trägerelement überragt und sich im eingesetzten Zustand im Inneren der im Knochen angebrachten Aussparung abstützt.

In den Figuren 2 und 3 sind Trägerelement 1 und Einsatzteil 5 für sich getrennt in perspketivischer Darstellung wiedergegeben. Die beiden Teile weisen aneinander angepaßte konisch geformte Bereiche 7 und 8 auf, welche mit einem gewissen Spiel behaftet sein können. Durch dieses Spiel kann die Elastizität des Ringes durch eine gerinfügige Verbiegung der Ringform wirksam werden. Die Krafteinleitung erfolgt über die Flanken des Kegelstumpfes 8. Der in Figur 3 wiedergegebene Einsatz 5 weist an seiner Unterseite eine Ausnehmung 16 zur Aufnahme des Kopfes der Femurschaftprothese auf. Aussparungen 17 bis 20 an einem Ansatz 21 ermöglichen zusammen mit einer Nase 22 am Trag ring 1 unterschiedliche Positionen des Einsatzes 5.

In Figur 2a ist als Ausschnitt aus der Figur 2 ein einzelnes Gewindesegment 2 wiedergegeben. Es ist ersichtlich, daß bei Voranbewegung in Pfeilrichtung zum Einschrauben der vorangeführte höchste Bereich des Segments 2 als Schneidwerkzeug dient, während der hintere Teil einen ausreichenden Freiwinkel aufweist und auf diese Weise auch ein Festklemmen des Rings beim Einschrauben sicher verhindert. Der "Rüken" des Gewindesegments wird bevorzugt durch ein-oder mehrfaches Uberfräsen erzeugt, so daß sich entsprechend eine mehreckige oder verrundete Struktur des Gewindesgements ergibt. Der beim Einschrauben vorangeführte Bereich entspricht dem vollständigen Gewindeprofil, während die nachgeführten Teile in ihrer maximalen Oberfläche zunehmend verringert sind.

Die Teile 1 und 5 sind ineinander schnappbar, wobei in eine entsprechende Rille des Einsatzteiles ein Drahtring 9 eingelegt ist, der in eine entsprechende Rille 10 des Trägerelementes 1 eingreift. Aussparungen 11 und 12 an der Unterseite des Trägerelementes diene zur Aufnahme eines Einschraubwerkzeuges, wobei durch die gleichmäßige Verteilung der Aussparungen (von denen zwei nicht sichtbar sind) ein exaktes Dirigieren während des Einschraubens möglich ist. Der kuppelförmig geformte Bereich 6 des Einsatzteiles 5 weist eine Strukturierung 13 auf, die mit der Knockenoberfläche in Kontakt kommt.

Die Einsätze stehen in unterschieldlichen Ausführungen zur Verfügung, wobei nach dem Einschrauben des Trägerteils 1 ein geeignetes Ensatzelement ausgewählt wird. Hierzu stehen geeignete Tiefenmeßelemente zur Verfügung, welche prinzipiell die Form des in Figur 3 dargestellten Einsatzes haben, wobei zusätzlich im Bereich der Kuppel eine (gestrichelt dargestellte) Aussparung 14 vorgesehen sein kann, welche beim Blick von unten durch den eingeschraubten Ring 1 hindurch eine Beurteilung zuläßt, ob der kuppelförmige Bereich 6 des Einsatzes 5 den Knochen erreicht. Die Aussparung 14 bildet damit eine Art "Fenster". Bei dem speziellen dieses Fenster aufweisenden Meßeinsatz-enfällt der Ring 9, wobei stattdessen bevorzugt auf dem zylindrischen Bereich Markierungen vorgesehen sind, welche in bezug auf die Kante zwischen zylindrischem und kegeligem Bereich des Einsatzes 1 angeben, welcher von mehreren Einsätzen im betreffenden Fall jeweils den besten Sitz bietet.

Es ist ersichtlich, daß die ringförmige Gestalt des Trägerelementes 1 einerseits für einen stabilen Sitz des Prothesenelementes (ohne Verwendung von Knochzement) in der Weise sorgt, daß die eigentliche Pfanne 5 präzise gehalten ist und nicht ihre einmal gefundene optimal justierte Position verlassen kann. Die Übertragung von bei Belastung der Prothese vorzugsweise auftretenden Druckkräfte erfolgt aber zum größten Teil über den kuppelförmigen Bereich des Einsatzes und zum anderen Teil auch über den kegeligen Ansatz, wobei mit diesem eine selbsttätige Zentrierung verbunden ist.

Der Ring gestattet beim Einschrauben infolge seiner inneren Öffnung einen großflächigen Durchblick auf den Innenbereich der Knockenaussparung, so daß zuverlässig beurteilt werden kann, wenn das Tragelement 1 beim Einschrau ben seine Endposition erreicht hat. Gerade bei sich verjüngenden Gewinden wird die volle Tragfähigkeit erst dann erreicht, wenn das Gewinde vollständig eingeschraupt ist und sich der Träger bündig an den Knochenhohlraum anschließt.

In der Figur 4 ist eine Ausführung eines Einsatzes mit unterschiedlichen Varianten von Aussparungen (gestrichelt) im Schnitt dargestellt, der zu dem erfindungsgemäßen System gehört, wobei alle Einsätze in ihren zylindrischen und konischen Außenbereich an den in den entsprechenden Figuren dargestellten ringförmigen Träger 1 angepaßt sind. Durch die unterschiedliche relative Lage der Ausnehmung 16 (Ausführungen 22' und 23 gestrichelt dargestellt) läßt sich jedoch der Drehpunkt für den Kopf der Schenkelhalsprothese entsprechend verlagern, so daß eine individuelle Anpassung möglich ist. Dabei ermöglicht die gestrichelt dargestellte Ausführung 22' eine unterschiedliche Höhenposition in bezug auf die Knockenoberfläche, während die gestrichelte Ausführung 23 für eine die exzentrische Aufnahme für den Prothesenkopf ausgebildet ist. Die Exentrizität kann durch Drehen des Einsatzes in eine geeignete Richtung gebracht werden, wobei die unterschiedlichen Raststellungen die jeweils eingestellte Position fixieren (Aussparungen 17 bis 20 in Figur 3 mit Nase 22 in Figur 2).

Bei einer bevorzugten nicht dargestellten Ausführungsform sind an dem Einsatz 5 in Figur 3 Erhebungen vorgesehen, welche in die Ausparungen 11, 12 für den Steckschlüssel am Einsatz 1 in Figur 2 angepaßt sind. Auf diese Weise ist eine Drehsicherung des Einsatzes 5 auch gewährleistet, wenn die Nase 22 entfällt. Die Aussparungen 11 und 12 erhalten damit eine Doppelfunktion.

Es sind weitere Einsätze möglich, welche unterschiedliche Höhen- und Achsenanordnungen miteinander kombinieren, so daß den auftretenden Anforderungen unter Berücksichtigung des in seiner optimalen Position beispielsweise bei Dysplasie eingeschraubten Trägerringes in jeder Weise Rechnung getragen werden kann. Eine gewünschte Neigung der Achsenrichtung wird nicht mittels eines speziellen Ein satzes, sondern über die Einschraubrichtung des Trägerelementes festgelegt, wie es nachfolgend dargestellt ist. Zur Aufnahme einer nicht dargestellten sogenannten "Doppelkopfprothese" ist die Ausnehmung 16, 22' oder 23 auf eine Kugel von der Größe der Femurkopf-Gelenkkugel aufschnappbar ausgebildet. Eine derartige "Doppelkopfprothese" bildet eine vollständige Hüftgelenkprothese, bei der die Außenabmessungen der Gelenkschale derjenigen der gesunden Gelenkkugel entsprechen.

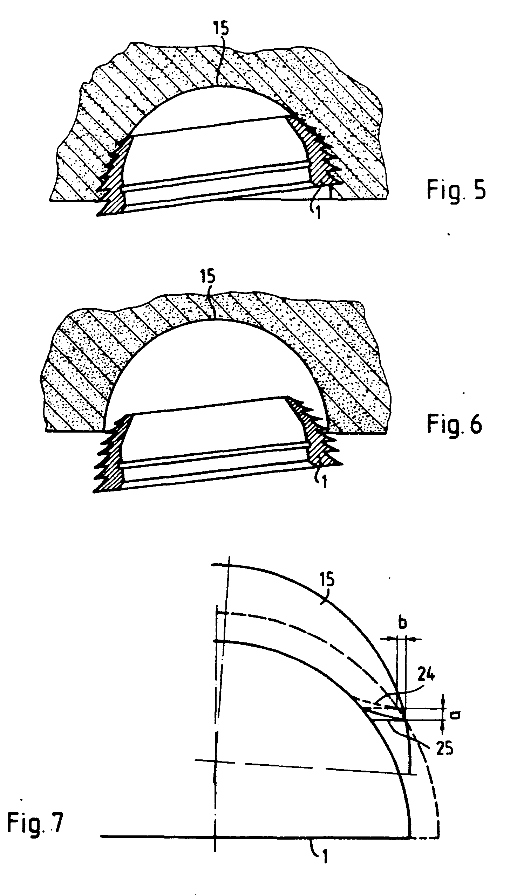

In den Figuren 5 und 6 ist bei den dortigen Schnittdarstellungen die Möglichkeit eines geneigten Einschraubens in eine Knochenexkavation 15 wiedergegeben. Es ist ersichtlich, daß bei dem selbstschneidenden Charakter des Gewindes in Verbindung mit der Oberfläche, die einen Teil einer Kugel bildet, verschiedene Positionen im halbkugelförmig ausgefrästen Knochenhohlraum erreicht werden können. Damit läßt sich das Tragelement entsprechend der Belastbarkeit des Knochens und der gewünschten Ausrichtung der Prothese einbringen. Die äußere Oberfläche des Kreisringes entspricht dabei der Oberfläche einer Kugelzone.

In Figur 6 ist schematisch dargestellt, wie das Einschneiden der Gewindesegmente des Trägeelementes in die Aussparung erfolgt.

Da der Radius der gefrästen Aussparung kleiner als der Radius der die Außenkanten der Gewindesegmente tangierenden Halbkugel und die Tangenten an die betreffenden Radien im Berührungspunkt nicht wie bei zylindrischen oder üblichen konischen Gewinden parallel verlaufen, sondern einen spitzen Winkel miteinander bilden und sich die beiden Kugelflächen in Richtung auf den Knochen noch zunehmend von einander entfernen, ist, da die Gewindesegmente eine gewisse Steigung aufweisen und der vorangeführte Teil sich in einem Bereich befindet, wo die beiden Kugelflächen ein ander nicht berühren, ohne die hier dargestellten Maßnahmen nicht sicherstellt, daß das Gewindesegment im Bereich der Schneidnut in die Knochenoberfläche eingreift. Die Gewindesegmente würden vielmehr mit ihrem Rücken zunächst mit der knochenoberfläche in Kontakt kommen und das Selbsteinschneiden des einzuschraubenden Ringes verhindern. Erst die entsprechende Abflachung der in Einschraubrichtung zurückliegenden Bereiche der Gewindesegmente schafft die notwendige Überhöhung des vorangeführten Teils, so daß die Schneidkanten bevorzugt punktförmig eingreifen können und somit auch den bei jedem Schneidwerkzeug notwendigen Freiwinkel aufweisen.

In Figur 7 sind in einer vergrößerten geometrischen Darstellung die Verhältnisse bei Einschraubengemäß Figur 6 wiedergegeben, wobei die beiden Dreiecke 24 und 25 auf dem inneren Umfang die Vorder- und Hinterkante eines Gewindesegments darstellen, dessen Rücken nicht, wie in Figur 2a dargestellt, angefast bzw. verrundet ist. Wegen der Steigung des Gewindesegments befinden sich die Schnittflächen gemäß Figur 7 in unterschiedlicher Höhe, wobei das obere (gestrichelt dargestellte) Dreieck 24 die beim Einschrauben vorangeführte Kante symbolisiert. Bei einer Höhendifferenz a infolge der Steigung befindet sich die vorangeführte (Schneid-) Kante um den Betrag b von der halbkugel förmigen Exkavation 15 entfernt, während die rückwärtige Fläche (unteres Dreieck 25) anliegt. Damit liegt das Gewindesegment mit seinem Rücken an der Knochenwandung an, ohne wie ein Schneidwerkzeug eindringen zu können. Die Folge ist, daß das Trägerelement nicht in die Knochenwandung zum Erzeugen des Gewindes einschneidet sondern dort abrutscht oder klemmt. Est ist damit ersichtlich, wie die vorgenommenen Anfasungen das Einschrauben ohne vorheriges Gewindeschneiden erleichtern insbesondere wenn dieses Einschrauben in eine halbkugelige Aussparung mit geneigter Achse erfolgen soll, wie es in Figur 6 wiedergegeben ist.

**Patentansprüche**

1. In den Knochen einschraubbares, ein selbstschneidendes Gewinde aufweisendes, ringförmig ausgebildetes Trägerelement (1) für eine künstliche Hüftpfanne mit Einsatz (5), dadurch gekennzeichnet, daß die die Gewindesegmente (2) tragende Außenoberfläche als Kugelzone ausgebildet ist, so daß das Trägerelement (1) in eine mittels eines Fräsers im Knochen erzeugte halbkugelförmige Aussparung in verschiedenen axialen Richtungen einschraubbar ist.

2. Trägerelement mit Einsatz nach Anspruch 1, dadurch gekennzeichnet, daß der Innendurchmesser des ringförmigen Trägerelements mindestens gleich seiner Höhe ist.

3. Trägerelement nach Anspruch 2, dadurch gekennzeichnet, daß Wandstärke und Elastizitätskonstante des Trägerelementes derart be messen sind, daß die elastische Verformbarkeit des Trägerelementes bei Belastung parallel zur Ringebene im wesentlichen derjenigen der umgebenden Knochenbereiche entspricht.

4. Trägerelement nach einem der vorangehenden Ansprüche dadurch gekennzeichnet, daß das Gewinde Schneidnuten (3) aufweist, wobei die in Einschraubrichtung vorangeführten Kanten (4) der den Schneidnuten benachbarten Gewindesegmente (2) die weiter zurückliegenden Bereiche derart überrangen, daß sie beim Einsetzen des Trägerelements in die halbkugelförmige Ausparung zum selbstschneidenden Einschrauben des Elementes mit der Knochenoberfläche eher in Eingriff kommen als die in Einschraubrichtung zurückliegenden Teile der Gewindesegmente.

5. Trägerelement nach einem der vorangehenden Ansprüche dadurch gekennzeichnet, daß eine umlaufende innere konische Anlagefläche (7) für den im eingeschraubten Zustand von außen her einsetzbaren Einsatz vorgesehen ist.

6. Trägerelement mit Einsatz nach einem der vorangehenden Ansprüche, dadurch gekennzeichnet, daß beim Einsatz die Aufnahme (23) für eine einzusetzende Femurkopfprothese exzentrisch angeordnet ist.

7. Trägerelement mit nach Anspruch 6, dadurch gekennzeichnet, daß Rasten (17 bis 20) zur drehsicheren Fixierung von Einsatz (5) und Trägerelement (1) in unterschiedlichen Winkelstellungen vorgesehen sind.

8. Trägerelement mit Einsatz nach einem der Ansprüche 1 bis 5, dadurch gekennzeichnet, daß die Stirnfläche (6) des Einsatzes (5) die Außenkontur des Trägerelements (1) zu einer Halbkugelform ergänszt.

9. Trägerelement mit Einsatz nach Anspruch 8, dadurch gekennzeichnet, daß die Stirnfläche des Einsatzes an ihrer Außenseite mit rechtwinklig zueinander gerichteten Rillen (13), versehen ist.

10. Trägerelement mit Einsatz nach einem der Ansprüche 1 bis 5, dadurch gekkenzeichnet, daß dieser zur unmittelbaren Aufnahme einer Doppelkopfprothese auf eine Gelenkkugel aufschnappbar ausgebildet ist.

**Revendications**

1. Elément de support (1) annulaire d'une cuvette de hanche artificielle avec pièce insérée ou insert (5), cet élément de support pouvant être vissé dans l'os et présentant un filetage autoarauder, caractérisé en ce que la surface extérieure qui porte les segments de filetage (2) est sous forme de segment sphérique de façon qui l'élément de support (1) puisse être vissé suivant différentes directions axiales dans un évidement hémisphérique creusé dans l'os au moyen d'une fraise.

2. Elément de support à insert selon la revendication 1, caractérisé en ce que le diamètre intérieur de l'élément de support annulaire est au moins égal à sa hauteur.

3. Elément de support selon la revendication 2, caractérisé en ce que l'épaisseur de paroi et la constante d'élastiticité de l'élément de support sont dimensionnées de manière que la déformabilité élastique de l'élément de support lors d'une mise en charge parallèlement au plan de l'anneau corresponde essentiellement à celle de la zone de l'os qui l'entoure.

4. Elément de support selon l'une des revendications précédentes, caractérisé en ce que le filetage présente des rainures de coupe (3), les arêtes (4) des segments de filetage (2) voisins des rainures de coupe, qui sont situées en avant suivant le direction de vissage, faisant saillie par rapport aux zones situées plus en amont d'une maînère telle que, lors de la mise en place de l'élément de support dans l'évidement de forme hémisphérique en vue du vissage autotaraudant de l'élément, elles viennent en prise avec la surface de l'os plus tôt que les parties des segments de filetage situées en amont suivant la direction de vissage.

5. Elément de support selon l'une des revendications précédentes, caractérisé en ce qu'il est prévu une surface d'appui conique interne faisant la tour (7) pour l'insert pouvant être mis en place de l'extérieur.

6. Elément de support à insert selon l'une des revendications précédentes, caractérisé en ce que le logement (23) pour une prothèse de tête de fémur à mettre en place est excentré lors de l'insertion.

7. Elément de support à insert selon la revendication 6, caractérisé en ce que des éléments d'arrêt (17 à 20) pour le blocage en rotation de l'insert (5) par rapport à l'élément de support (1) sont prévus à différentes positions angulaires.

8. Elément de support à insert selon l'une des revendication 1 à 5, caractérisé en ce que la surface frontale (6) de l'insert (5) complète le contour extérieur de l'élément de support (1) pour donner une forme hémisphérique.

9. Elément de support à insert selon la revendication 8, caractérisé en ce que la surface frontale de l'insert, sur son côté extérieur, est munie de gorges (13) orientées perpendiculairement entre elles.

10. Elément de support à l'insert selon l'une des revendications 1 à 5, caractérisé en ce qu'il est agencé, pour la réception directe d'une prothèse à double tête, de mainière à pouvoir être enclenché automatiquement sur une sphère articulaire.

## Claims

1. A supporting element (1), which is of annular construction and which can be screwed into the bone and having a self-tapping screw-thread, for an artificial acetabulum with insert (5), characterised in that the outer surface which carries the thread segments (2) has a spherical region, so that the support element (1) can be screwed in various axial directions into a hemispherical recess produced by means of a milling cutter in the bone.

2. A supporting element as claimed in Claim 1, characterised in that the internal diameter of the annular element is at least equal to its height.

3. A supporting element as claimed in Claim 2, characterised in that the wall thickness and elasticity constant of the support element are such that the resilient deformability of the supporting element on loading parallel to the plane of the ring corresponds substantially to that of the surrounding bone regions.

4. A supporting element as claimed in one of the preceding Claims, characterised in that the screw-thread comprises cutting grooves (3) and the leading edges (4), in the screw-in direction, of the screw-thread segments (2) adjacent to the cutting grooves project above the regions situated further back, so that when the supporting element is inserted in the hemispherical recess for self-tapping screw insertion of the element, they come into engagement with the surface of the bone earlier than the parts of the screw-thread segments situated further back in the screw-in direction.

5. A supporting element as claimed in one of the preceding Claims, characterised in that a circular internal tapered contact surface (7) is provided for the insert which can be inserted from the outside in the screwed-in state.

6. A supporting element with insert as claimed in one of the preceding Claims, characterised in that on the insert the receiver (23) for an artificial femur head to be inserted is disposed eccentrically.

7. A supporting element with insert as claimed in Claim 6, characterised in that detents (17 to 20) are provided to locate insert (5) and supporting element (1) so as to hold them against rotation in different angular positions.

8. A supporting element with insert as claimed in one of the Claims 1 to 5, characterised in that the end face (6) of the insert (5) supplements the external contour of the supporting element (1) to form a hemispherical shape.

9. A supporting element with insert as claimed in Claim 8, characterised in that the end face of the insert is provided with furrows (13) directed at right angles to one another.

10. A supporting element with insert as claimed in one of the Claims 1 to 5, characterised in that it is adapted to snap onto the spherical part of a ball-and-socket joint in order to receive a double-headed prosthesis directly.

Fig.1

Fig.2a

Fig. 2

Fig.3

Fig.4

Fig. 5

Fig. 6

Fig. 7